# EUROPEAN PATENT SPECIFICATION

(11) **EP 2 464 400 B1**
(45) Date of publication and mention of the grant of the patent: **28.01.2015**
(21) Application number: 10740637.3
(22) Date of filing: 05.08.2010
(51) Int. Cl.: A61M 5/32

(54) **Cap for a portable medical delivery device and such a medical delivery device**
Kappe für eine tragbare medizinische Abgabevorrichtung und eine derartige medizinische Abgabevorrichtung
Bouchon pour dispositif d'administration médicale portable et ce dispositif d'administration médicale

(30) Priority: 12.08.2009 EP 09008851
(43) Date of publication of application: 20.06.2012
(73) Proprietor: Sanofi-Aventis Deutschland GmbH, 65929 Frankfurt am Main (DE)
(72) Inventor: BRÜGGEMANN, Ulrich, 65926 Frankfurt am Main (DE); TUBB, Andrew, Surrey GU1 4YS (GB); LANGLEY, Christopher, Warwickshire CV32 7HH (GB); TYCE, Daniel, Rugby Warwickshire CV22 5RY (GB)
(86) International application number: PCT/EP2010/061421
(87) International publication number: WO 2011/018408

(56) References cited:
- WO-A1-2007/036676
- FR-A1- 2 876 034
- GB-A- 2 438 593
- US-A- 5 829 589
- US-A- 5 980 495
- US-A1- 2005 027 259
- US-B1- 6 398 762

## Description

The present invention relates to a cap for a portable medical delivery device and such a medical delivery device.

Particularly, the present invention relates to a cap for a portable medical delivery device, having an axial direction defining a first end and a second end of the cap comprising an opening for receiving at least a front part of a medical delivery device defined at the first end of the cap and a socket for covering a needle mounting portion of the medical delivery device.

Such devices are used for instance by patients suffering from the disease of diabetes. This disease necessitates the application of an ingredient, known as insulin, several times a day, especially before the patient enjoys his meal. Therefore the patient carries along with him a portable medical delivery device, also known as an insulin syringe. These devices typically contain a housing in which a receptacle or a cartridge comprising the insulin is integrated, as well as a dosage unit for regulating the dosage of the ingredient injected at each time the syringe is used. On the housing a low prized exchangeable needle is mounted for injecting the insulin under the skin of the patient. Such needles are, however, only usable for a predetermined number of injections and then have to be exchanged, so that the patients have to carry spare needles with them. For carrying spare needles along additional cases attachable to the medical delivery device are known in the prior art.

The patent application US 2008/0108951 A1 shows a storage system for needles pen delivery device syringe and a hypodermic needle adapted for attachment to and use with a pen delivery device syringe. Such a system, however, has the problem that the case for storing the spare needle is an additional part of the medical delivery device and must be separately mounted on the delivery device. Therefore on the one hand the number of parts during the production process of the medical delivery device is increased and on the other hand there remains the danger that the patient may forget or even lose the additional part.

From EP 0 927 056 B1 a syringe comprising a cap being located beside the needle mounted on the housing ready for use, the cap including a compartment in which at least one needle can be stored. With the provision of such a cap an additional part for providing a compartment for storing a needle is necessary which is uncomfortable for a user. Further, this solution is ergonomically disadvantageous as there might occur confusion for a patient in certain state of health as to which cap might be the right one to be used for injecting the medicament of the syringe.

From US 2005 0027259 A1, a prefilled hypodermic syringe is known. Such syringe is connected to one single needle which is protected by a cap. The cap comprises an inner rubber liner for receiving the mounted needle. However, the syringe of this document still encounters the problem that no spare needles can be stored at the device or the cap itself. Similar technical teachings as to one single cap for one single needle already mounted to a body can also be derived from US 5 980 495 A, US 6 398 762 B1 and FR 2 876 034 A1.

It an object of the invention to provide a portable medical delivery device by which the use of the syringe is ergonomically helpful to a user with regard to the use of the needle of the syringe which must be removed from the syringe after an injection or which must be mounted before a next injection. A further object of the invention is to provide corresponding parts of the syringe in order to provide means for a better handling of a spare needle in an ergonomic way.

These objects are solved by a cap providing the feature of the independent claims. Further embodiments and examples of the invention are shown in the dependent claims which are referred back thereto.

According to an aspect of the invention, a cap for a portable medical delivery device is provided, comprising a needle for dispensing a pharmaceutical formulation therethrough in a distal direction, a front part and a needle mounting portion for mounting the needle, the cap having an axial direction defining a first, distal end and a second, proximal end and comprising:
- an opening at the first end of the cap for receiving the front part of the medical delivery device,
- a socket for receiving the needle mounting portion of the medical delivery device. In the interior of the cap at least one receptacle is formed which is designed for storing at least one needle in the interior of the cap, the receptacle having a bottom lying at the second end of the cap and an opening lying a distance extending from the bottom towards the first end of the cap.

With such an embodiment the spare needles can be stored directly in the cap of the portable medical delivery device and therefore be always attached to the device itself, such that no additional storing members are needed.

At least one receptacle of the cap can be designed for storing at least one spare needle.

According to this embodiment the sterile, unused, spare needles can easily be locked in a first receptacle, such that when the patient takes off the cap no sterile needle may fall out hazardously and therefore be damaged or get lost.

According to an example of the invention, the cap can comprise an opening and closing device which is disposed at the opening of the first receptacle, wherein the closing device comprises a cover which is moveable between a first position in which the opening is closed and a second position in which the opening is opened and vice versa, wherein the closing device is designed such that in its closed position a spare needle is kept inside the first receptacle and in its opened position a spare needle can be inserted in the first receptacle or can be removed from the first receptacle.

In a further embodiment of the present invention the cap may further comprise a second receptacle being designed for storing at least one used needle. In particular, in the interior of the cap comprises a receptacle being designed for storing at least one used needle, wherein the receptacle comprises a covering device disposed at an opening of the second receptacle and an opening and closing device which is moveable between a first position in which the opening is at least partly covered and a second position in which the opening is opened. In this example, the socket can be disposed between a first and a second receptacle with regard to the axial direction of the cap.

Generally, the covering device of the second receptacle being designed for storing at least one used needle can be realized such that a used needle can be introduced into the second receptacle but not removed from the second receptacle via the covering device by a user. In particular, the covering device can comprise a cover which is designed such that a used needle can be pressed through by a user such that the removal of the used needle through the same cover by the user is practically not possible. By this measure, the user will not take a used needle by mistake. In this regard, the covering device can be made of an elastic material and comprises an opening over which an elastic cover extends and which comprises a slot through which a used needle can be pressed. Further, the covering device can be designed such that the covering device or the cover can be removed from the opening. In this regard, the cover can be designed as flexible cover so that it can easily be mounted for example on an upper part or elevated edge of the receptacle or removed therefrom.

According to a further example of the invention, the receptacle comprising the covering device has a first end disposed at the first end of the cap and a second end disposed at a distance in the axial direction towards the first end of the cap, wherein the covering device is disposed at the first end of the cap.

According to an alternative example of the invention, the receptacle comprising the covering device has a first end disposed at the first end of the cap and a second end disposed at a distance in the axial direction towards the first end of the cap, wherein the covering device is disposed at the second end of the cap.

According to an alternative example of the invention, the receptacle comprising the covering device has a first end disposed at the first end of the cap and a second end disposed at a distance in the axial direction towards the first end of the cap, wherein the covering device is disposed at a side opening which extends from the inside of the portion of the receptacle comprising the covering device and a side wall of the cap. According to a further example of the invention, the receptacle comprising the covering device has a first end disposed at the first end of the cap and a second end disposed at a distance in the axial direction towards the first end of the cap, wherein the receptacle comprising the covering device comprises an opening and opening and closing device disposed at the opening by which the covering device is moveable between a first position in which the opening is at least partly covered and a second position in which the opening is opened.

In this aspect of the invention, the opening at which the opening and closing device is disposed can be located at the first end of the receptacle.

Alternatively, the opening at which the opening and closing device is disposed can be located at the second end of the receptacle and wherein the opening and closing device comprises the covering device and wherein the opening and closing device is designed such that the covering device can be moved between an opened and a closed position.

As a further alternative, the opening at which the opening and closing device is disposed is lying at a side opening of the cap which extends from the inside of the portion of the receptacle comprising the covering device and a side wall of the cap. With regard to the opening and closing device, the cap can comprise a locking device which is moveable between a locking state for locking the opening and closing device and a releasing state for opening the opening and closing device.

According to an example of the invention, the locking device comprises an operating element which is located at least partially outside the cap.

Generally, the locking device can be designed as a flexible element. Alternatively, the locking device can be designed as a latching device.

According to a further aspect of the invention, the locking device and/or the first receptacle being designed for storing at least one used needle, if any, and/or the second receptacle comprising the covering device, if any, is marked by a colour. In particular, the two receptacles of the cap can comprise coloured surfaces, wherein at least one surface of a first receptacle has a different colour than at least one surface of the other receptacle.

By using colours according to the invention, the user can securely and quickly distinguish visually the receptacle providing spare needles from the receptacle providing used needles and therefore confusion is prevented.

According to a further aspect of the invention, a medical delivery device can be provided, comprising a housing having an needle mounting portion for mounting a needle on the housing and a receptacle for storing a medicament to be delivered via the needle when being mounted on the needle mounting portion, wherein the needle mounting portion can be covered by a cap according to one embodiment or example of the invention. In particular, the needle mounting portion can be designed such that the needle can be detachably mounted on the needle mounting portion.

The above mentioned features may be combined partly or as a whole.

The term "medical delivery device" or "drug delivery device" according to instant invention shall mean a single-dose or multi-dose or pre-set dose or pre-defined dose, disposable or re-useable device designed to dispense a user selectable or pre-defined dose of a medicinal product, preferably multiple pre-defined doses, e.g. insulin, growth hormones, low molecular weight heparins, and their analogues and/or derivatives etc. Said device may be of any shape, e.g. compact or pen-type. The medication delivery device can be a pen-type device or an injector-type device. Furthermore, the said device may comprise a needle or may be needle-free. In addition, the said device may comprise a fixed needle or a replaceable needle or a moving needle or a shielded moving needle. In particular, the term "medical delivery device" shall mean a disposable needle-based pen-type device providing multiple pre-defined doses having mechanical and manual dose delivery and dose selection mechanisms, which is designed for use by persons without formal medical training such as patients. Preferably, the drug delivery device is of the injector-type.

The medical delivery device comprises a drive mechanism for dispensing a set of a dose of a medicament from a medication receptacle of the delivery device in a distal direction. Dose selection or dose setting for selection or for setting a dose of a liquid or non-liquid medicament in a receptacle may be provided through a dosing mechanism or dose setting means or a dose setting mechanism so that the medical delivery device can further comprise a dose selection mechanism or dose setting mechanism for selection or setting the dose of a liquid medicament in the receptacle to be dispensed by the drive mechanism. The dosing mechanism can partly or totally be realized with components or functions of the drive mechanism or can be realized as a separate mechanism.

The term "distal end" according to instant invention shall mean the end of the device or a component of the device which is closest to the dispensing end of the device. The term "proximal end" according to instant invention shall mean the end of the device or a component of the device which is furthest away from the dispensing end of the device. Accordingly, the "distal direction" is the direction which is directed from the proximal end to the distal end of the device or a component of the device and the "proximal direction" is the direction which is directed from the distal end to the proximal end of the device or a component of the device.

Dose delivery may be provided through a mechanical (optionally manual) drive mechanism or drive means or electrical drive mechanism or electro-mechanical mechanism or stored energy drive mechanism, such as a spring, etc.. The drive mechanism can in particular comprise a piston being movable in the medication receptacle for displacing the medicament in the receptacle. At the piston a piston rod may be attached

The term "housing" according to instant invention shall preferably mean any exterior housing ("main housing", "body", "shell") or interior housing ("insert", "inner body") having one or more helical threads. The housing may be designed to enable the safe, correct, and comfortable handling of the drug delivery device or any of its mechanism. Usually, it is designed to house, fix, protect, guide, and/or engage with any of the inner components of the drug delivery device (e.g., the drive mechanism, cartridge, plunger, piston rod) by limiting the exposure to contaminants, such as liquid, dust, dirt etc. In general, the housing may be unitary or a multipart component of tubular or non-tubular shape. Usually, the exterior housing serves to house a cartridge, which may be replaceable or non-replaceable, from which a number of doses of a medicinal product may by dispensed.

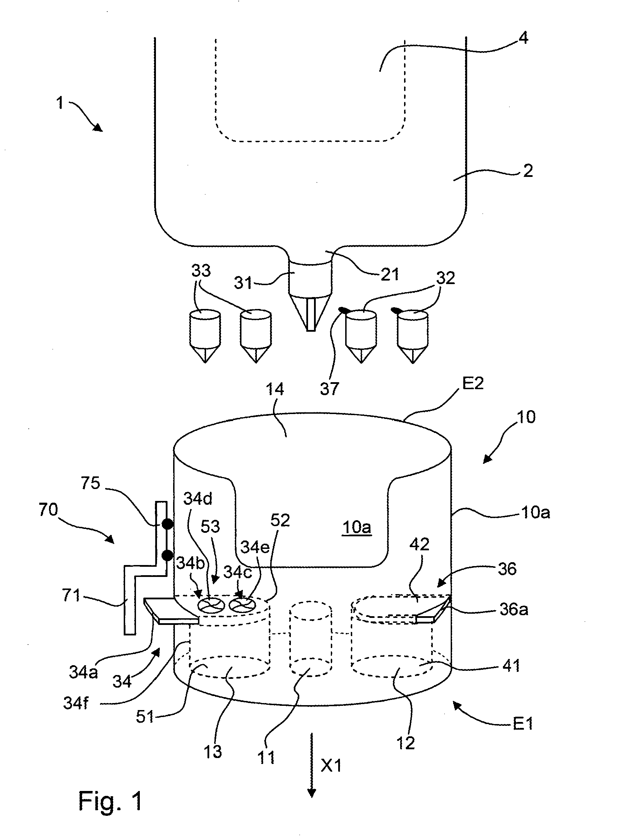

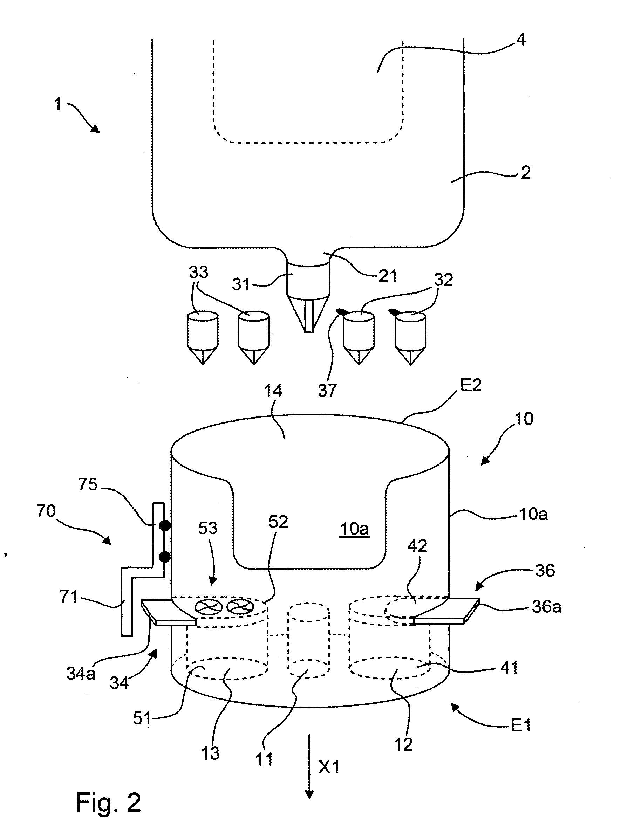

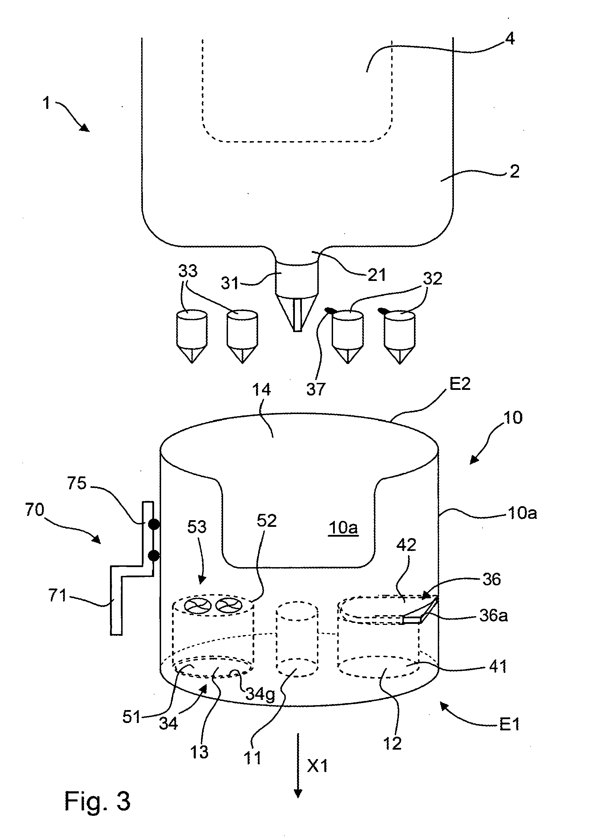

The invention will be understood in greater details from the following description of the preferred embodiments thereof, which are given only by way of example and with reference to the accompanying drawing, in which:
▪ Fig. 1 is a perspective side view of a medical delivery device including an embodiment of a cap according to the present invention, the cap having a socket for receiving a needle mounting portion of the medical delivery device, one receptacle for storing at least one spare needle comprising a cover which is shown in a closed position and one receptacle for storing at least one used needle comprising a covering device which is disposed in the inner space of the cap,
▪ Fig. 2 is a perspective side view of the medical delivery device including the embodiment of a cap according to figure 1, wherein the cover of the receptacle for storing at least one spare needle is shown in an opened position,
▪ Fig. 3 is a perspective side view of the medical delivery device including the embodiment of a cap according to figure 1, wherein the cover of the receptacle for storing at least one used needle is disposed at the distal end of the cap.

Referring to the drawing in Fig. 1 a housing 2 is shown, which comprises a cartridge 4 in which a medicament for injection is retained. The cartridge 4 is detachably mounted in the housing 2 and can particularly be adapted for dispensing a plurality of insulin dosages during a predetermined period like for instance one day. When activated the dosage is controlled by a driving mechanism and a dosing mechanism (not shown in the figures).

The housing 2 has a needle mounting portion 21 for receiving a needle 31. In the figures the needle 31 is provided with a storage cover which has to be taken away before injection.

In the figures, also spare needle assemblies 32 are shown in an embodiment which comprises a protecting cover having each a film 37 for closing the protecting cover. Before using the spare needle a patient has to take off the film 37 to take the new needle out off its cover.

Furthermore in Fig. 1 a cap 10 is shown which is of an essentially cylindrical shape having a first end or distal end E1 and a second end or proximal end E2 in its axial or longitudinal direction X1. At the first end of the cap an opening 14 is provided for receiving a front part of the medical delivery device 20 which comprises a needle mounting portion 21 is provided. At the second end the cap 10 is essentially closed. In an interior space or interior 10a of the cap 10 a socket 11 is provided for receiving the needle mounting portion 21 of the housing 2, wherein the socket 11 is designed for receiving the needle mounting portion 21 without needle mounted thereon and/or or a needle mounting portion 21 on which a needle 31 is mounted. The socket 11 can particularly be designed such that it may only receive the needle mounting portion 21, whereby the user is forced to remove a needle 31 used for an injection after each injection before mounting the cap 10 on the housing 2.

In the interior space 10a of the cap 10 a first receptacle 12 is disposed in which spare needles 32 or packages including spare needles may be introduced. The first receptacle has a first end or bottom 41 lying at a first or the distal end E1 of the cap 10 and an opening 43 lying at a second or proximal end 42 in a distance extending from the bottom 41 towards the second end E2 or the proximal end of the cap 10. At or on the opening 42 an opening and closing device 36 comprising a cover 36a is disposed which can be moved between an opened and a closed position. In figure 1 the cover 36a is shown in a closed position and in figure 2 the cover 36a is shown in an opened position. In an opened state of the first closing unit spare needles can be introduced or removed from the first receptacle 12. The opening and closing device 36 is operable by a user from the outside of the cap 10.

The first receptacle 12 may be designed such that spare needles 32 or packages including spare needles may only be contained in a predetermined orientation inside the receptacle 12. The opening 43 can be oriented towards the opening 14 (figures 1 to 3) or can be disposed at the distal end E1 of the cap 10.

Alternatively or in addition, in the interior of the cap 10 a second receptacle 13 can be provided for receiving or for storing at least one used needle 33. The receptacle 12 can particularly comprise a covering device 34a which is disposed at an opening 53 of the second receptacle 12 and an opening and closing device 34 by which the covering device 34a is moveable between a first position in which the opening 53 is at least partly covered and a second position in which the opening 53 is opened. The opening 53 is located at the end of the receptacle 13 which is disposed at a distance from the distal end E1 towards the proximal end E2 of the cap 10.

The covering device 34a is designed such that a used needle 33 can be introduced into the second receptacle 13 but not removed from the second receptacle via the covering device 50 by a user. The covering device 34a according to the example shown in figure 1 comprises two openings 34b, 34c each of which are covered by a cover 34d, 34e which both are designed such that a used needle 33 can be pressed through by a user and such that the needle 33 which is inserted in the receptacle 13 can practically not be removed therefrom by the user.

In the embodiment shown in the figures, the covers 34d, 34e have a circular shape with at least two flaps and slots radially extending between the flaps. The flaps fold into the interior of the second receptacle 13 when a user pushes a used needle 33 through the covers 34d, 34e. However, the flaps are made of elastic material and designed such that they cannot be folded to the opposite side, being an outside of the second receptacle 13, so that it is not possible to pull a used needle out of the second receptacle 13 through the second closing unit 34, once it has been pushed inside.

By moving the covering device 34a together with the covers 34d, 34e in its opened position, the receptacle is opened for removal of the needles from the receptacle 13. Such a covering device 34a with at least one cover 34d, 34e can alternatively be disposed at an opening which is disposed at the distal end 51 or at a side wall 34f of the receptacle.

Alternatively, the covering device 34a with at least one combination of an opening and a cover extending over the same can be fixedly disposed on the proximal end of the receptacle 13. In this embodiment, a further cover 34g can be disposed on the distal end or a side wall 34f of the receptacle 13 (figure 3).

In the embodiment shown in figures 1 and 2, the socket 11 is disposed between the first and the second receptacle 12, 13 with regard to the axial direction X1 of the cap 10.

The opening and closing device 34 can be locked by a locking device 70 or latching device by means of an operating element 71, can be operated by a user. One embodiment of the locking device 70 is shown in the figures 1 to 3 in a locking state. For discharging the second receptacle 13 the user has to pull out the locking device 70 in a direction which is directed away from the cap 10 such that the covering device 34a can be moved in an opened position so that the used needles can be removed from the second receptacle 13 for example by the gravity force. The locking device 70 is provided at one side portion of the cap 10. For safety reasons, before pulling out the opening and closing device 34 the user has to move the locking unit 35 into an unlocked position.

Alternatively, the operating element 71 can be disposed by a guiding device 75 at a side wall of the cap by which the operating element 71 can be moved between an opened and a closed position by a linear movement.

For example, the receptacles 12, 13 and/or the opening and closing device 34 and/or the covering device 34a and/or the cover 36a can be marked by colours which in particular can differ from each other. Thereby, the user can easily identify the type of receptacle. Also, the opening and closing device 34 and the locking device 70 can be marked by the same colour

The above mentioned features or embodiments of the present invention may be combined partly or as a whole in any way.

## Claims

1. Cap (10) for a portable medical delivery device (1) comprising a needle for dispensing a pharmaceutical formulation therethrough in a distal direction, a front part and a needle mounting portion (21) for mounting the needle, the cap (10) having an axial direction (X1) defining a distal end (E1) and a proximal end (E2) and comprising:
- an opening (14) at the distal end (E1) of the cap (10) for receiving the front part (2) of the medical delivery device (1),
- a socket (11) for receiving the needle mounting portion (21) of the medical delivery device (1),
**characterized in that**
- in the interior (10a) of the cap (10) at least one first receptacle (12) is formed which is designed for storing at least one spare needle (32) in the interior (10a) of the cap (10), the first receptacle (12) having a bottom (41) lying at the distal end (E1) of the cap (10) and an opening (42) lying at a distance extending from the bottom (41) towards the proximal end (E2) of the cap (10).

2. Cap (10) according to claim 1, wherein an opening and closing device (36) is disposed at the opening (41) of the first receptacle (12), wherein the closing device (36) comprises a cover (36a) which is moveable between a first position in which the opening (41) is closed and a second position in which the opening (41) is opened and vice versa, wherein the closing device (36) is designed such that in its closed position a spare needle (32) is kept inside the first receptacle (12) and in its opened position a spare needle (32) can be inserted in the first receptacle (12) or can be removed from the first receptacle (12).

3. Cap (10) according to one of claims 1 or 2, wherein in the interior (10a) of the cap (10) comprises a second receptacle (13) being designed for storing at least one used needle (33), wherein the second receptacle (13) comprises a covering device (34a) disposed at an opening (53) of the second receptacle (13) and an opening and closing device (34) which is moveable between a first position in which the opening (53) is at least partly covered and a second position in which the opening (53) is opened.

4. Cap (10) according to claim 2, wherein the covering device (34a) of the second receptacle (13) is designed such that a used needle (33) can be introduced into the second receptacle (13) but not removed from the second receptacle (13) via the covering device (34a) by a user.

5. Cap (10) according to claim 4, wherein the second receptacle (13) comprises an opening (52) over which a cover extends which is designed such that a used needle (33) can be pressed through by a user.

6. Cap (10) according to claim 5, wherein the covering device (34a) is made of an elastic material and comprises an opening over which an elastic cover extends and which comprises a slot through which a used needle (33) can be pressed.

7. Cap (10) according to one of the claims 4 to 6, wherein the second receptacle (13) comprising the covering device (34a) has a first end (51) disposed at the distal end (E1) of the cap (10) and a second end (52) disposed at a distance in the axial direction (X1) towards the distal end (E1) of the cap (10), wherein the covering device (34a) is disposed at the distal end (E1) of the cap (10).

8. Cap (10) according to one of the claims 4 to 6, wherein the second receptacle (13) comprising the covering device (34a) has a first end (51) disposed at the distal end (E1) of the cap (10) and a second end (52) disposed at a distance in the axial direction (X1) towards the distal end (E1) of the cap (10), wherein the covering device (34a) is disposed at the proximal end (E2) of the cap (10).

9. Cap (10) according to one of the claims 4 to 6, wherein the second receptacle (13) comprising the covering device (34a) has a first end (51) disposed at the distal end (E1) of the cap (10) and a second end (52) disposed at a distance in the axial direction (X1) towards the distal end (E1) of the cap (10), wherein the covering device (34a) is disposed at a side opening which extends from the inside of the portion of the second receptacle (13) comprising the covering device (34a) and a side wall (10a) of the cap (10).

10. Cap (10) according to claim 4 to 9, wherein the second receptacle (13) comprising the covering device (34a) has a first end (51) disposed at the distal end (E1) of the cap (10) and a second end (52) disposed at a distance in the axial direction (X1) towards the distal end (E1) of the cap (10), wherein the second receptacle (13) comprising the covering device (34a) comprises an opening and opening and closing device (34) disposed at the opening (53) by which the covering device (34a) is moveable between a first position in which the opening (53) is at least partly covered and a second position in which the opening (53) is opened.

11. Cap (10) according to claim 10, wherein the opening (53) at which the opening and closing device (34) is disposed is lying at the first end (51) of the second receptacle (13).

12. Cap (10) according to claim 11, wherein the opening (53) at which the opening and closing device (34) is disposed is lying at the second end (52) of the second receptacle (13) and wherein the opening and closing device (34) comprises the covering device (34a) and wherein the opening and closing device (34) is designed such that the covering device (34a) can be moved between an opened and a closed position.

13. Cap (10) according to claim 12, wherein the opening (53) at which the opening and closing device (34) is disposed is lying at a side opening of the cap (10) which extends from the inside of the portion of the second receptacle (13) comprising the covering device (34a) and a side wall (10a) of the cap (10).

14. Cap (10) according to one of claims 10 to 13, wherein the cap (10) comprises a locking device (70) which is moveable between a locking state for locking the opening and closing device (34) and a releasing state for opening the opening and closing device (34).

15. Medical delivery device (1), comprising a housing (2) having an needle mounting portion (3) for mounting a needle on the housing (2) and a receptacle (4) for storing a medicament to be delivered via the needle (31) when being mounted on the needle mounting portion (21), **characterized in that** the needle mounting portion (3) is covered by a cap (10) according to one of the preceding claims.

## Patentansprüche

1. Kappe (10) für eine tragbare medizinische Abgabevorrichtung (1), die eine Nadel zur Ausgabe einer pharmazeutischen Formulierung dort hindurch in einer distalen Richtung, ein Vorderteil und einen Nadelbefestigungsabschnitt (21) zur Befestigung der Nadel umfasst, wobei die Kappe (10) eine axiale Richtung (X1) aufweist, die ein distales Ende (E1) und ein proximales Ende (E2) definiert, und umfassend:
- eine Öffnung (14) an dem distalen Ende (E1) der Kappe (10) zur Aufnahme des Vorderteils (2) der medizinischen Abgabevorrichtung (1),
- eine Buchse (11) zur Aufnahme des Nadelbefestigungsabschnitts (21) der medizinischen Abgabevorrichtung (1),
**dadurch gekennzeichnet, dass**
- in dem Innenraum (10a) der Kappe (10) zumindest ein erster Aufnahmebehälter (12) geformt ist, der zur Aufbewahrung zumindest einer Ersatznadel (32) in dem Innenraum (10a) der Kappe (10) konstruiert ist, wobei der erste Aufnahmebehälter (12) eine Unterseite (41), die am distalen Ende (E1) der Kappe (10) liegt, und eine Öffnung (42) aufweist, die sich in einem Abstand von der Unterseite (41) zu dem proximalen Ende (E2) der Kappe (10) erstreckt.

2. Kappe (10) nach Anspruch 1, worin eine Öffnungs- und Schließvorrichtung (36) an der Öffnung (41) des ersten Aufnahmebehälters (12) angeordnet ist, worin die Schließvorrichtung (36) eine Abdeckung (36a) umfasst, die zwischen einer ersten Stellung, in der die Öffnung (41) geschlossen ist, und einer zweiten Stellung, in der die Öffnung (41) geöffnet ist, bewegt werden kann und umgekehrt, worin die Schließvorrichtung (36) so konstruiert ist, dass in ihrer geschlossenen Stellung eine Ersatznadel (32) in dem ersten Aufnahmebehälter (12) gehalten wird und in ihrer geöffneten Stellung eine Ersatznadel (32) in den ersten Aufnahmebehälter (12) eingeführt werden kann oder aus dem ersten Aufnahmebehälter (12) entfernt werden kann.

3. Kappe (10) nach einem der Ansprüche 1 oder 2, worin der Innenraum (10a) der Kappe (10) einen zweiten Aufnahmebehälter (13) umfasst, der zur Aufbewahrung zumindest einer gebrauchten Nadel (33) konstruiert ist, worin der zweite Aufnahmebehälter (13) eine Abdeckvorrichtung (34a), die an einer Öffnung (53) des zweiten Aufnahmebehälters (13) angeordnet ist, und eine Öffnungs- und Schließvorrichtung (34) umfasst, die zwischen einer ersten Stellung, in der die Öffnung (53) zumindest teilweise abgedeckt ist, und einer zweiten Stellung, in der die Öffnung (53) geöffnet ist, bewegt werden.

4. Kappe (10) nach Anspruch 2, worin die Abdeckvorrichtung (34a) des zweiten Aufnahmebehälters (13) so konstruiert ist, dass eine gebrauchte Nadel (33) in den zweiten Aufnahmebehälter (13) eingeführt werden kann, aber über die Abdeckvorrichtung (34a) von einem Anwender nicht aus dem zweiten Aufnahmebehälter (13) entfernt werden kann.

5. Kappe (10) nach Anspruch 4, worin der zweite Aufnahmebehälter (13) eine Öffnung (52) umfasst, über die sich eine Abdeckung erstreckt, die so konstruiert ist, dass eine gebrauchte Nadel (33) von einem Anwender durchgedrückt werden kann.

6. Kappe (10) nach Anspruch 5, worin die Abdeckvorrichtung (34a) aus einem elastischen Material gefertigt ist und eine Öffnung umfasst, über die sich eine elastische Abdeckung erstreckt und die einen Schlitz umfasst, durch eine gebrauchte Nadel (33) gedrückt werden kann.

7. Kappe (10) nach einem der Ansprüche 4 bis 6, worin der zweite Aufnahmebehälter (13) mit der Abdeckvorrichtung (34a) ein erstes Ende (51), das an dem distalen Ende (E1) der Kappe (10) angeordnet ist, und ein zweites Ende (52) aufweist, das in einem Abstand in der axialen Richtung (X1) zu dem distalen Ende (E1) der Kappe (10) angeordnet ist, worin die Abdeckvorrichtung (34a) an dem distalen Ende (E1) der Kappe (10) angeordnet ist.

8. Kappe (10) nach einem der Ansprüche 4 bis 6, worin der zweite Aufnahmebehälter (13) mit der Abdeckvorrichtung (34a) ein erstes Ende (51), das an dem distalen Ende (E1) der Kappe (10) angeordnet ist, und ein zweites Ende (52) aufweist, das in einem Abstand in der axialen Richtung (X1) zu dem distalen Ende (E1) der Kappe (10) angeordnet ist, worin die Abdeckvorrichtung (34a) an dem proximalen Ende (E2) der Kappe (10) angeordnet ist.

9. Kappe (10) nach einem der Ansprüche 4 bis 6, worin der zweite Aufnahmebehälter (13) mit der Abdeckvorrichtung (34a) ein erstes Ende (51), das an dem distalen Ende (E1) der Kappe (10) angeordnet ist, und ein zweites Ende (52) aufweist, das in einem Abstand in der axialen Richtung (X1) zu dem distalen Ende (E1) der Kappe (10) angeordnet ist, worin die Abdeckvorrichtung (34a) an einer Seitenöffnung angeordnet ist, die sich von der Innenseite des Abschnitts des zweiten Aufnahmebehälters (13) mit der Abdeckvorrichtung (34a) und einer Seitenwand (10a) der Kappe (10) erstreckt.

10. Kappe (10) nach einem der Ansprüche 4 bis 9, worin der zweite Aufnahmebehälter (13) mit der Abdeckvorrichtung (34a) ein erstes Ende (51), das an dem distalen Ende (E1) der Kappe (10) angeordnet ist, und ein zweites Ende (52) aufweist, das in einem Abstand in der axialen Richtung (X1) zu dem distalen Ende (E1) der Kappe (10) angeordnet ist, worin der zweite Aufnahmebehälter (13) mit der Abdeckvorrichtung (34a) eine Öffnung und eine Öffnungs- und Schließvorrichtung (34) umfasst, die an der Öffnung (53) angeordnet ist, wodurch die Abdeckvorrichtung (34a) zwischen einer ersten Stellung, in der die Öffnung (53) zumindest teilweise abgedeckt ist, und einer zweiten Stellung, in der die Öffnung (53) geöffnet ist, bewegt werden kann.

11. Kappe (10) nach Anspruch 10, worin die Öffnung (53), an der die Öffnungs- und Schließvorrichtung (34) angeordnet ist, an dem ersten Ende (51) des zweiten Aufnahmebehälters (13) liegt.

12. Kappe (10) nach Anspruch 11, worin die Öffnung (53), an der die Öffnungs- und Schließvorrichtung (34) angeordnet ist, an dem zweiten Ende (52) des zweiten Aufnahmebehälters (13) liegt und worin die Öffnungsund Schließvorrichtung (34) die Abdeckvorrichtung (34a) umfasst und worin die Öffnungs- und Schließvorrichtung (34) so konstruiert ist, dass die Abdeckvorrichtung (34a) zwischen einer geöffneten und einer geschlossenen Stellung bewegt werden kann.

13. Kappe (10) nach Anspruch 12, worin die Öffnung (53), an der die Öffnungs- und Schließvorrichtung (34) angeordnet ist, an einer Seitenöffnung der Kappe (10) liegt, die sich von der Innenseite des Abschnitts des zweiten Aufnahmebehälters (13) mit der Abdeckvorrichtung (34a) und einer Seitenwand (10a) der Kappe (10) erstreckt.

14. Kappe (10) nach einem der Ansprüche 10 bis 13, worin die Kappe (10) eine Verriegelungsvorrichtung (70) umfasst, die zwischen einem Verriegelungszustand zur Verriegelung der Öffnungs- und Schließvorrichtung (34) und einem Freigabezustand zur Öffnung der Öffnungs- und Schließvorrichtung (34) bewegt werden kann.

15. Medizinische Abgabevorrichtung (1), umfassend ein Gehäuse (2) mit einem Nadelbefestigungsabschnitt (21) zur Befestigung einer Nadel auf dem Gehäuse (2) und einen Aufnahmebehälter (4) zur Aufbewahrung eines Medikaments, das durch die Nadel (31) abgegeben werden soll, wenn diese an dem Nadelbefestigungsabschnitt (21) befestigt ist, **dadurch gekennzeichnet, dass** der Nadelbefestigungsabschnitt (21) von einer Kappe (10) nach einem der vorhergehenden Ansprüche abgedeckt wird.

## Revendications

1. Bouchon (10) pour un dispositif d'administration médical portable (1) comprenant une aiguille pour distribuer une formulation pharmaceutique à travers elle dans une direction distale, une partie avant et une portion de fixation d'aiguille (21) pour fixer l'aiguille, le bouchon (10) ayant une direction axiale (X1) définissant une extrémité distale (E1) et une extrémité proximale (E2) et comprenant :
- une ouverture (14) à l'extrémité distale (E1) du bouchon (10) pour recevoir la partie avant (2) du dispositif d'administration médical (1),
- une douille (11) pour recevoir la portion de fixation d'aiguille (21) du dispositif d'administration médical (1),
**caractérisé en ce que**
- au moins un premier réceptacle (12) est formé à l'intérieur (10a) du bouchon (10), lequel premier réceptacle est conçu pour stocker au moins une aiguille de rechange (30) à l'intérieur (10a) du bouchon (10), le premier réceptacle (12) ayant un fond (41) situé à l'extrémité distale (E1) du bouchon (10) et une ouverture (42) située à une certaine distance du fond (41) vers l'extrémité proximale (E2) du bouchon (10).

2. Bouchon (10) selon la revendication 1, dans lequel un dispositif d'ouverture et de fermeture (36) est disposé au niveau de l'ouverture (41) du premier réceptacle (12), le dispositif de fermeture (36) comprenant un couvercle (36a) qui peut être déplacé entre une première position dans laquelle l'ouverture (41) est fermée et une deuxième position dans laquelle l'ouverture (41) est ouverte et vice versa, le dispositif de fermeture (36) étant conçu de telle sorte que dans sa position fermée, une aiguille de rechange (32) soit conservée à l'intérieur du premier réceptacle (12) et que dans sa position ouverte, une aiguille de rechange (32) puisse être insérée dans le premier réceptacle (12) ou puisse être retirée du premier réceptacle (12).

3. Bouchon (10) selon l'une des revendications 1 ou 2, dans lequel, à l'intérieur (10a) du bouchon (10) est compris un deuxième réceptacle (13) conçu pour stocker au moins une aiguille usagée (33), le deuxième réceptacle (13) comprenant un dispositif de recouvrement (34a) disposé au niveau d'une ouverture (53) du deuxième réceptacle (13) et un dispositif d'ouverture et de fermeture (34) qui peut être déplacé entre une première position dans laquelle l'ouverture (53) est au moins en partie couverte et une deuxième position dans laquelle l'ouverture (53) est ouverte.

4. Bouchon (10) selon la revendication 2, dans lequel le dispositif de recouvrement (34a) du deuxième réceptacle (13) est conçu de telle sorte qu'une aiguille usagée (33) puisse être introduite dans le deuxième réceptacle (13) mais ne puisse pas être retirée du deuxième réceptacle (13) par le biais du dispositif de recouvrement (34a) par un utilisateur.

5. Bouchon (10) selon la revendication 4, dans lequel le deuxième réceptacle (13) comprend une ouverture (52) par-dessus laquelle s'étend un couvercle qui est conçu de telle sorte qu'une aiguille usagée (33) puisse être enfoncée à travers lui par un utilisateur.

6. Bouchon (10) selon la revendication 5, dans lequel le dispositif de recouvrement (34a) est fabriqué en un matériau élastique et comprend une ouverture, par-dessus laquelle s'étend un couvercle élastique, et qui comprend une fente à travers laquelle peut être enfoncée une aiguille usagée (33).

7. Bouchon (10) selon l'une des revendications 4 à 6, dans lequel le deuxième réceptacle (13) comprenant le dispositif de recouvrement (34a) présente une première extrémité (51) disposée à l'extrémité distale (E1) du bouchon (10) et une deuxième extrémité (52) disposée à une certaine distance dans la direction axiale (X1) vers l'extrémité distale (E1) du bouchon (10), le dispositif de recouvrement (34a) étant disposé au niveau de l'extrémité distale (E1) du bouchon (10).

8. Bouchon (10) selon l'une des revendications 4 à 6, dans lequel le deuxième réceptacle (13) comprenant le dispositif de recouvrement (34a) présente une première extrémité (51) disposée à l'extrémité distale (E1) du bouchon (10) et une deuxième extrémité (52) disposée à une certaine distance dans la direction axiale (X1) vers l'extrémité distale (E1) du bouchon (10), le dispositif de recouvrement (34a) étant disposé au niveau de l'extrémité proximale (E2) du bouchon (10).

9. Bouchon (10) selon l'une des revendications 4 à 6, dans lequel le deuxième réceptacle (13) comprenant le dispositif de recouvrement (34a) présente une première extrémité (51) disposée à l'extrémité distale (E1) du bouchon (10) et une deuxième extrémité (52) disposée à une certaine distance dans la direction axiale (X1) vers l'extrémité distale (E1) du bouchon (10), le dispositif de recouvrement (34a) étant disposé au niveau d'une ouverture latérale qui s'étend depuis l'intérieur de la portion du deuxième réceptacle (13) comprenant le dispositif de recouvrement (34a) et une paroi latérale (10a) du bouchon (10).

10. Bouchon (10) selon les revendications 4 à 9, dans lequel le deuxième réceptacle (13) comprenant le dispositif de recouvrement (34a) présente une première extrémité (51) disposée à l'extrémité distale (E1) du bouchon (10) et une deuxième extrémité (52) disposée à une certaine distance dans la direction axiale (X1) vers l'extrémité distale (E1) du bouchon (10), le deuxième réceptacle (13) comprenant le dispositif de recouvrement (34a) comprenant une ouverture et un dispositif d'ouverture et de fermeture (34) disposé au niveau de l'ouverture (53) grâce auquel le dispositif de recouvrement (34a) peut être déplacé entre une première position dans laquelle l'ouverture (53) est au moins en partie couverte et une deuxième position dans laquelle l'ouverture (53) est ouverte.

11. Bouchon (10) selon la revendication 10, dans lequel l'ouverture (53) au niveau de laquelle le dispositif d'ouverture et de fermeture (34) est disposé se situe au niveau de la première extrémité (51) du deuxième réceptacle (13).

12. Bouchon (10) selon la revendication 11, dans lequel l'ouverture (53) au niveau de laquelle est disposé le dispositif d'ouverture et de fermeture (34) est située au niveau de la deuxième extrémité (52) du deuxième réceptacle (13) et dans lequel le dispositif d'ouverture et de fermeture (34) comprend le dispositif de recouvrement (34a) et dans lequel le dispositif d'ouverture et de fermeture (34) est conçu de telle sorte que le dispositif de recouvrement (34a) puisse être déplacé entre une position ouverte et une position fermée.

13. Bouchon (10) selon la revendication 12, dans lequel l'ouverture (53) au niveau de laquelle est disposé le dispositif d'ouverture et de fermeture (34) est située au niveau d'une ouverture latérale du bouchon (10) qui s'étend depuis l'intérieur de la portion du deuxième réceptacle (13) comprenant le dispositif de recouvrement (34a) et une paroi latérale (10a) du bouchon (10).

14. Bouchon (10) selon l'une des revendications 10 à 13, dans lequel le bouchon (10) comprend un dispositif de verrouillage (70) qui peut être déplacé entre un état de verrouillage pour verrouiller le dispositif d'ouverture et de fermeture (34) et un état de libération pour ouvrir le dispositif d'ouverture et de fermeture (34).

15. Dispositif d'administration médical (1), comprenant un boîtier (2) ayant une portion de fixation d'aiguille (21) pour fixer une aiguille sur le boîtier (2) et un réceptacle (4) pour stocker un médicament devant être distribué par le biais de l'aiguille (31) lorsqu'elle est fixée sur la portion de fixation d'aiguille (21), **caractérisé en ce que** la portion de fixation d'aiguille (21) est recouverte par un bouchon (10) selon l'une quelconque des revendications précédentes.
